Europäisches Patentamt

European Patent Office

Office européen des brevets

(19)

(11) Publication number: **0 123 416**
**A1**

# (12) EUROPEAN PATENT APPLICATION

(21) Application number: **84301865.6**

(22) Date of filing: **20.03.84**

(51) Int. Cl.³: **C 07 C 53/122**
**C 07 C 51/41**

(30) Priority: **26.03.83 GB 8308384**

(43) Date of publication of application:
**31.10.84 Bulletin 84/44**

(84) Designated Contracting States:
**BE DE FR GB IT NL**

(71) Applicant: **Verdugt b.v.**
**Papesteeg 91**
**NL-4006 WC Tiel(NL)**

(72) Inventor: **van Ooyen, Johannes Adrianus Cornelis**
**Verdugt b.v. Papesteeg 91**
**NL-4006 WC Tiel(NL)**

(74) Representative: **Krishnan, Suryanarayana**
**Kalyana et al,**
**c/o The British Petroleum Company plc Patents Division**
**Chertsey Road**
**Sunbury-on-Thames Middlesex TW16 7LN(GB)**

(54) **Method of producing solid potassium dipropionate.**

(57) The present invention relates to a process for producing solid potassium dipropionate by mixing an aqueous solution of neutral potassium propionate with an equimolar proportion of propionic acid and cooling the mixture. In the process propionic acid is mixed with the aqueous solution containing at least 85% w/w of the neutral propionate at a temperature above 30°C, the mixture is cooled to a temperature of 30°C or less to enable the solid dipropionate to crystallise and the dipropionate is thereafter recovered. The solid potassium dipropionate can be used as a preservative for human foods and in animal feedstuffs.

EP 0 123 416 A1

Croydon Printing Company Ltd.

1

## METHOD OF PRODUCING SOLID POTASSIUM DIPROPIONATE

The present invention relates to a method of producing solid potassium dipropionate.

Potassium dipropionate, also known as potassium monohydrogen propionate is a complex acid salt containing one mole of propionate ion and one mole of propionic acid per mole of potassium ion. Aqueous solutions of potassium dipropionate and the use thereof as a preservative is claimed and described in British Patent Specification No 1505388. Solid acid salts are more difficult to prepare because of their inherent instability. For instance sodium dipropionate rapidly loses a mole of propionic acid when exposed to atmosphere leaving the neutral, sodium propionate salt behind. Moreover, the acid salts when produced are not free flowing unless precautions are taken during preparation and storage of the complex acid salts.

GB 1473447 discloses a method of producing complex acid salts of this type by preparing a fluidised bed of the solid neutral salt and spraying the solid neutral salt in the fluidised bed with the free acid. The process is cumbersome because it involves using vapours of the free acid which is relatively corrosive and also possesses an unpleasant odour.

It has now been found that problems involving the use of free acid vapours may be mitigated by using a combination of solutions which is subsequently cooled to allow crystallisation of the complex acid salts.

Accordingly, the present invention is a process for producing solid potassium dipropionate said process comprising mixing an aqueous solution of neutral potassium propionate with an equimolar proportion of propionic acid and cooling the mixture, characterised in that the propionic acid is mixed with the aqueous solution containing at least 85% w/w of the neutral propionate at a temperature above 30°C, the mixture is cooled to a temperature of 30°C or less to enable the solid dipropionate to crystallise and the dipropionate is thereafter recovered.

The aqueous solution containing more than 85% w/w, preferably from 85 to 90% w/w of the neutral propionate is preferably prepared by dissolving neutral potassium propionate which is free of any water of crystallisation either in water or in a relatively dilute aqueous solution of the neutral propionate.

The neutral potassium propionate free from water of crystallisation is a desirable starting material because potassium propionate normally crystallises with one mole of water of crystallisation and such crystals are difficult to redissolve in water unless high temperatures are used. Moreover direct evaporation of dilute aqueous solutions of the neutral propionate to produce a concentrated aqueous solution containing more than 85% w/w of the neutral salt is difficult because at about 80% concentration the solution becomes viscous and the neutral salt monohydrate begins to separate which is difficult to redissolve. Moreover, the viscosity of the solution increases sharply thereby making continued stirring and evaporation of the solution difficult. Hence the use of a neutral salt free of water of crystallisation is preferred.

The neutral salt free from water of crystallisation may be prepared by spray drying an aqueous solution of the neutral salt. The relatively dilute aqueous solution used for this purpose suitably has a neutral salt content of less than 85% w/w preferably from 40 to 60% w/w, most preferably from 45 to 55% w/w. The neutral salt so produced though slightly moist is substantially free of water of crystallisation and dissolves easily in water. Thus to produce an aqueous solution containing more than 85% w/w of the neutral salt, the solid neutral salt free of water of crystallisation may be dissolved either as such in water to derive the appropriate concentration or may be mixed with the appropriate amount of a previously prepared relatively dilute aqueous solution of the neutral salt which was used for the spray drying stage.

The concentrated aqueous solution of the neutral salt so produced and having a neutral salt content of above 85% w/w is then mixed with an equimolar amount of propionic acid at a temperature above 30°C, typically from 55 to 65°C. When the mixing is complete, the resultant mixture is cooled down to a temperature of 30°C or less, preferably from 20 to 30°C thereby enabling solid, crystalline potassium dipropionate to separate. The product dipropionate is thereafter separated and recovered by conventional techniques.

The product thus produced is a crystalline solid, which is free flowing and is stable. The solid potassium dipropionate can be used as a preservative, either alone or admixed with other adjuvants in human foods and in animal feedstuffs.

The present invention is further illustrated with reference to the following Example.

Example

Solid crystalline potassium dipropionate was produced as follows:

(a)  Aqueous potassium hydroxide (50% w/w solution) was reacted with propionic acid to form an aqueous solution of neutral potassium propionate (50% w/w).

(b)  A portion of the solution from (a) above was spray dried to form neutral potassium propionate powder substantially free from water of crystallisation.

(c)  The solid, powder form (b) of the neutral salt was mixed with the remaining aqueous solution from (a) after step (b) at 90°C so as to form a concentrated aqueous solution of neutral potassium propionate (86% w/w).

(d)  To the concentrated aqueous solution in (c) was added an equimolar proportion of propionic acid and thoroughly mixed at about 60°C.

(e)    The mixture from (d) was cooled down to about 30°C when crystals of potassium dipropionate separated. The crystals were recovered by centrifugation and drying.

Case 5558(2)

CA 01234 16

Claims:

1. A process for producing solid potassium dipropionate said process comprising mixing an aqueous solution of neutral potassium propionate with an equimolar proportion of propionic acid and cooling the mixture, characterised in that the propionic acid is mixed with the aqueous solution containing at least 85% w/w of the neutral propionate at a temperature above 30°C, the mixture is cooled to a temperature of 30°C or less to enable the solid dipropionate to crystallise and the dipropionate is thereafter recovered.

2. A process according to claim 1 wherein the aqueous solution contains from 85 to 90% w/w of the neutral propionate.

3. A process according to claims 1 or 2 wherein the aqueous solution of the neutral propionate is prepared by dissolving neutral potassium propionate which is free of any water of crystallisation either in water or in a relatively dilute aqueous solution of the neutral propionate.

4. A process according to claim 3 wherein the neutral potassium propionate free of water of crystallisation is prepared by spray drying a relatively dilute aqueous solution of the neutral propionate.

5. A process according to claims 3 or 4 wherein the relatively dilute aqueous solution of the neutral propionate contains less than 85% w/w of the neutral propionate.

6. A process according to claim 5 wherein the relatively dilute aqueous solution of the neutral propionate contains from 40 to 60% w/w of the neutral propionate.

7. A process according to any one of the preceding claims wherein the propionic acid is mixed with the aqueous solution containing at least 85% w/w of the neutral propionate at a temperature from 55 to 65°C.

8. A process according to any one of the preceding claims wherein the mixture of propionic acid and the aqueous solution containing at least 85% w/w of the neutral propionate is cooled to a temperature from 20 to 30°C thereby enabling solid crystalline potassium dipropionate to separate.

# EUROPEAN SEARCH REPORT

**EUROPEAN PATENT OFFICE**

0123416

Application number

EP 84 30 1865

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | EP-A-0 001 488 (BP)<br>* Claims 1-4 *<br><br>--- | 1 | C 07 C 53/122<br>C 07 C 51/41 |
| A | EP-A-0 032 807 (BP)<br>* Claim 4; example 1 *<br><br>--- | 1 | |
| A,D | GB-A-1 473 447 (HOECHST)<br>* Claim 1 *<br><br>----- | 1 | |

TECHNICAL FIELDS SEARCHED (Int. Cl. ³)

C 07 C 51/41
C 07 C 53/00
C 07 C 53/122

The present search report has been drawn up for all claims

| Place of search | Date of completion of the search | Examiner |
|---|---|---|
| BERLIN | 22-06-1984 | KNAACK M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82